(19) Europäisches Patentamt
European Patent Office
Office européen des brevets

(11) Veröffentlichungsnummer: **0 332 970**
**A1**

(12) **EUROPÄISCHE PATENTANMELDUNG**

(21) Anmeldenummer: **89103879.6**

(22) Anmeldetag: **06.03.89**

(51) Int. Cl.⁴: **B01J 31/16 , C07C 51/56**

(30) Priorität: **17.03.88 DE 3808868**

(43) Veröffentlichungstag der Anmeldung:
**20.09.89 Patentblatt 89/38**

(84) Benannte Vertragsstaaten:
**AT BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Luft, Gerhard, Prof.Dr.**
**Ludwigstrasse 141a**
**D-6109 Mühltal(DE)**
Erfinder: **Trabold, Peter, Dr.**
**Ahornweg 19a**
**D-6110 Dieburg(DE)**

(54) **Trägerkatalysator für die Herstellung von Monocarbonsäureanhydriden.**

(57) In einem Trägerkatalysator für die Herstellung von Monocarbonsäureanhydriden durch Carbonylierung der entsprechenden Ester oder Ether, worin eine Organosiliciumverbindung als mehrfunktioneller Haftvermittler einerseits an ein Trägermaterial und andererseits an eine Edelmetallverbindung aus der Gruppe VIII des Periodensystems der Elemente gebunden ist, ist der Haftvermittler eine chelatbildende Organosiliciumverbindung der allgemeinen Formel

EP 0 332 970 A1

a)

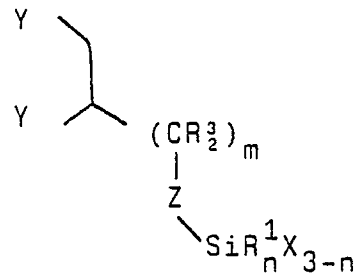

oder

b)

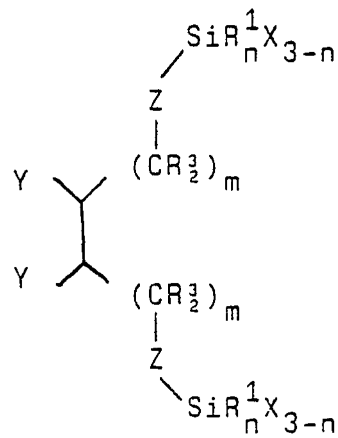

wobei
X = -Cl, -Br oder $-OR^2$;
Y = $-NR^4_2$, ein stickstoffhaltiger Arylrest, $-PR^4_2$, $AsR^4_2$, $-SR^4$ oder -SH;
Z = null, Arylen, Phenylen (ggf. ortho-, meta- oder parasubstituiert)
$R^1$ = $C_1$ bis $C_5$-Alkyl;
$R^2$ = $C_1$ bis $C_5$-Alkyl oder $-C_6H_5$; $R^3$ = -H, $C_1$ bis $C_3$-Alkyl; $R^4$ = $C_1$ bis $C_6$-Alkyl, $C_5$ oder $C_6$-Cycloalkyl oder $-C_6H_5$ oder $-CH_2C_6H_5$, die ggf. mit Halogen-, Methoxy-, Ethoxy- oder $C_1$ bis $C_3$-Alkylgruppen substituiert sind;
n = 0 oder 1 oder 2;
m = 2 bis 6, vorzugsweise 2 bis 4.

## Trägerkatalysator für die Herstellung von Monocarbonsäureanhydriden

Die Erfindung betrifft einen Trägerkatalysator für die Herstellung von Monocarbonsäureanhydriden durch Carbonylierung der entsprechenden Ester oder Ether, worin eine Organosiliciumverbindung mit Alkoxy- oder Halogengruppen sowie mit Organostickstoff-, Organophosphor-, Organoarsen-, Organoschwefel- oder Mercaptogruppen als mehrfunktioneller Haftvermittler einerseits an ein Trägermaterial und andererseits an eine Edelmetallverbindung aus der Gruppe VIII des Periodensystems der Elemente gebunden ist.

Ein solcher Trägerkatalysator ist aus der DE-Offenlegungsschrift 34 40 646 bekannt. Außerdem beschreibt die DE-OS 35 11 048 A1 einen Trägerkatalysator, in dem das Trägermaterial mit der Lösung einer Edelmetallchelatverbindung, die aus der Edelmetallverbindung und einem Chelator mit Organostickstoff-, Organophosphor-, Organoarsen-, Organoschwefel- oder Mercaptogruppen gebildet ist, lediglich imprägniert wurde.

Es ist Aufgabe der vorliegenden Erfindung, den Chelator so zu modifizieren, daß er als mehrfunktioneller Haftvermittler wirkt und sich Standzeit (Aktivitätsdauer) und Selektivität des Trägerkatalysators bei gleichem Trägermaterial deutlich verbessern

Der erfindungsgemäße Trägerkatalysator ist insbesondere bestimmt für die Herstellung von Monocarbonsäureanhydriden der allgemeinen Formel $(RCO)_2O$ durch Umsetzung eines Carbonsäureesters oder Dialkylethers der allgemeinen Formeln RCOOR bzw. ROR, wobei R jeweils denselben Alkylrest mit 1-4 C-Ato men bedeutet, mit Kohlenmonoxid in der Gasphase in Gegenwart von Jod oder Brom oder deren Verbindungen als Reaktionspromotor bei Temperaturen von 130 bis 400 °C und Drücken von 1-150 bar.

Ein derartiges, in der Gasphase mit einem Trägerkatalysator arbeitendes Verfahren ist bereits aus der DE-Offenlegungsschrift 34 40 647 bekannt, welche die bei den Flüssigphaseverfahren auftretenden Nachteile, z.B. die schwierige Abtrennung und Rückführung von suspendiertem und teilweise gelöstem Katalysator und ggf. Promotor, vermeidet.

Im einzelnen ist die Erfindung dadurch gekennzeichnet, daß der Haftvermittler eine chelatbildende Organosiliciumverbindung der allgemeinen Formel

a) 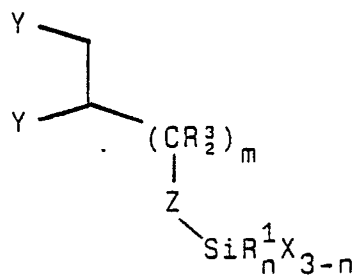 oder

b) 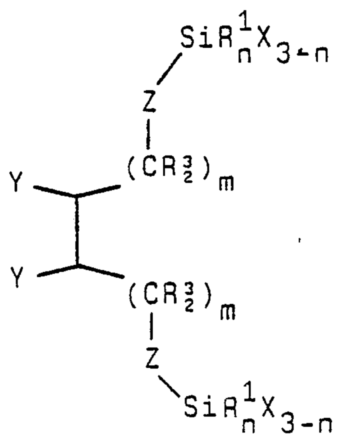

ist,
wobei

X = -Cl, -Br oder $-OR^2$;
Y = $-NR^4_2$, ein stickstoffhaltiger Arylrest, $-PR^4_2$, $AsR^4_2$, $-SR^4$ oder -SH;
Z = null, Arylen, Phenylen (ggf. ortho-, meta- oder parasubstituiert)
$R^1$ = $C_1$ bis $C_5$-Alkyl;
$R^2$ = $C_1$ bis $C_5$-Alkyl oder $-C_6H_5$; $R^3$ = -H, $C_1$ bis $C_3$-Alkyl; $R^4$ = $C_1$ bis $C_6$-Alkyl, $C_5$ oder $C_6$-Cycloalkyl oder $-C_6H_5$ oder $-CH_2C_6H_5$, die ggf. mit Halogen-, Methoxy-, Ethoxy- oder $C_1$ bis $C_3$-Alkylgruppen substituiert sind;
n = 0 oder 1 oder 2;
m = 2 bis 6, vorzugsweise 2 bis 4.

Der Trägerkatalysator der Erfindung kann weiterhin wahlweise und bevorzugt dadurch gekennzeichnet sein, daß

a) in ihm die chelatbildende Organosiliciumverbindung als mehrfunktioneller Haftvermittler einerseits an das Trägermaterial und andererseits abwechselnd an die Edelmetallverbindung und an eine Nichtedelmetallverbindung aus der 6. oder 8. Nebengruppe des Periodensystems gebunden ist.

b) er zusätzlich Nichtedelmetallverbindungen aus der 1. bis 3. Hauptgruppe oder der 4. bis 6. oder der 8. Nebengruppe des Periodensystems als Promotoren enthält.

c) er ein anorganisches oxidisches Trägermaterial oder einen Aktivkohleträger enthält, deren restliche aktive Hydroxygruppen durch Veresterung oder Veretherung desaktiviert wurden.

d) er zusammen 0,01 bis 50 Gew%, vorzugsweise 0,1 bis 20 Gew%, Edelmetallverbindung, Haftvermittler und ggf. Nichtedelmetallverbindung enthält.

Als Katalysatorträger kommen bevorzugt anorganische Oxide, wie z.B. $SiO_2$, $Al_2O_3$, MgO, $TiO_2$, $La_2O_3$, $ZrO_2$, Zeolith, Ton, NiO, $Cr_2O_3$, $WO_3$ oder entsprechende Mischoxide, aber auch Aktivkohle in Frage, welche BET-Oberflächen von 1-1000 $m^2/g$, vorzugsweise 30-400 $m^2/g$, haben und stets noch aktive OH-Gruppen aufweisen müssen. Diese OH-Gruppen reagieren mit der oder den funktionellen Gruppen X des Haftvermittlers unter Bildung von Sauerstoffbrücken zwischen Träger und Haftvermittler.
Ebenso wie gemäß DE-OS 34 40 646 und 35 11 048 sind die Promotoren der 5. oder 6. Hauptgruppe in den erfindungsgemäß eingesetzten Haftvermittlern chemisch gebunden. Sie bilden selbst eine funktionelle Gruppe, die die Edelmetallverbindungen aus der Gruppe VIII und ggf. Nichtedelmetallverbindungen aus der 6. oder 8. Nebengruppe chelatisieren.

Es ist ein Vorteil, daß die zur Steigerung der Aktivität und der Selektivität der erfindungsgemäßen Trägerkatalysatoren notwendigen Promotoren aus der 5. oder 6. Hauptgruppe des Periodensystems der Elemente eine funktionelle Gruppe Y in den mehrfunktionellen Haftvermittlern bilden und somit bis zur maximalen Konzentration, die durch die Anzahl der OH-Gruppen auf der Trägeroberfläche bestimmt ist, fixiert werden können. Deshalb entfällt eine Abtrennung und Rückführung dieser z.B. Organostickstoff- oder Organophosphorpromotoren.

Der Trägerkatalysator der Erfindung für die Herstellung von Monocarbonsäureanhydriden weist gegenüber den Trägerkatalysatoren der DE-OS 34 40 646 höhere Selektivitäten und besonders bei Langzeitbelastung höhere Standzeiten auf.

Ein weiterer Vorteil der Erfindung ist darin zu sehen, daß es möglich ist, Edelmetallchelate chemisch auf Oberflächen von Trägermaterialien zu fixieren. Weiterhin zeigen die auf dem Trägermaterial aufgetragenen modifizierten Edelmetall-Chelatverbindungen und ggf. Nichtedelmetall-Chelatverbindungen noch höhere Schmelzpunkte (240 - 270 °C) als die in den DE-OS 34 40 646 und 35 11 048 beschriebenen Komplexe, was zu einer höheren thermischen Stabilität der Katalysatoren bzw. zu einer Erhöhung des Anwendungsbereichs von 20 bis 50 °C führt.

Der Trägerkatalysator der Erfindung dient insbesondere zur Herstellung von Essigsäureanhydrid aus Methylacetat oder Dimethylether in Gegenwart von Methyljodid oder Methylbromid als Reaktionspromotor. Als Reaktionspromotor kann auch HI, HBr oder allgemein RI oder RBr eingesetzt werden, wobei R einen Alkylrest mit 1-4 C-Atomen darstellt.

In den allgemeinen Formeln für die Organosiliciumverbindungen, welche als Haftvermittler (Spacer) in Frage kommen, bedeutet X vorzugsweise $-OR^2$ und insbesondere Methoxy oder Ethoxy. Sofern n nicht null ist, bedeutet $R^1$ einen unverzweigten Alkylrest, insbesondere Methyl, Ethyl oder Propyl.

Die Trägermaterialien wurden bereits genannt; als Mischoxide kommen z.B. $Cr_2O_3$ - $Al_2O_3$, $WO_3$ - $Al_2O_3$, MgO - $Al_2O_3$, $SiO_2$ -$Al_2O_3$ oder $ZrO_2$ - $Al_2O_3$ in Frage. Der Trägerkatalysator enthält bevorzugt 0.05 bis 5 Gew% Edelmetall.

Als Edelmetallverbindungen können bei der Herstellung des Trägerkatalysators z.B. folgende Verbindungen eingesetzt werden:

Rhodium:

$RhCl_3$, $RhCl_3 . 3 H_2O$, $RhBr_3$, $RhI_3$, $Rh(NO_3)_3$, $Rh_2(CO)_4Cl_2$, $Rh_2(CO)_4Br_2$, $Rh(CO)_4I_2$, $[P(C_6H_5)_3]_3RhCl$, $[P-(C_6H_5)_3]_2Rh(CO)Cl$, $Rh_6(CO)_{16}$, $Rh_4(CO)_{12}$, $Rh_2(O_2CCH_3)_4$, $[RhCl(C_8H_{12})]_2$;

Iridium:

$IrCl_3$, $[Ir(CO)_3Cl]_2$, $Ir[P(C_6H_5)_3]_2(CO)Cl$, $Ir_4(CO)_{12}$, $[IrCl(C_8H_{12})]_2$, $Cl(CO)_2Irpyr$ (pyr = $C_6H_5N$);

Palladium:

$PdCl_2$, $PdBr_2$, $PdI_2$, $(CH_3CO_2)_2Pd[P(C_6H_5)_3]_2$, $PdCl_2[P(C_6H_5)_3]_2$, $Pd(O_2CCH_3)_2$, $PdCl_2(C_8H_{12})$, $(C_6H_5CN)-_2PdCl_2$;

Ruthenium:

$RuCl_3$, $Ru_3(CO)_{12}$, $RuCl_2[P(C_6H_5)_3]_3$, $RuCl_2(CO)_2[P(C_6H_5)_3]_2$, $[RuCl_2(CO)_3]_2$.

Als Nichtedelmetallverbindungen aus der 6. oder 8. Nebengruppe, insbesondere Cr, Ni, aber auch W, Fe, Co, die ebenfalls mit den Chelatoren reagieren, kommen z.B. ferner in Frage:

Chrom:

$Cr(CO)_6$, $CrCl_3$, $C_7H_8Cr(CO)_3$.

Nickel:

$Ni(CO)_4$, $[P(C_6H_5)_3]_2Ni(CO)_2$, $NiCl_2$, $Ni(C_8H_{12})_2$.

Als Nichtedelmetallverbindungen aus der 1. bis 3. Hauptgruppe oder der 4. bis 6. oder der 8. Nebengruppe des Perioden-systems, vorzugsweise des Li, Na, Mg, Ca, Al, Ti, Zr, V, Cr, W, Fe, Co, Ni, können z.B. Hydroxide, Carbonate, Carbonyle, Hydride, Halogenide und andere Salze eingesetzt werden. Diese Verbindungen unedler Metalle können zusätzlich, z. B. als. Lösung durch Imprägnierung, auf den Katalysatorträger aufgebracht werden.

Zur Herstellung des Trägerkatalysators der Erfindung muß zuerst der mehrfunktionelle Haftvermittler, d.h. die chelatbildende Organosiliciumverbindung, mit den funktionellen Gruppen Y bereitgestellt werden. Dieser kann nach oder in Analogie zu Literaturangaben dargestellt werden. Im all gemeinen wird dann eine der genannten Edelmetallverbindungen aus der Gruppe VIII und ggf. eine der genannten Nichtedelmetallverbindungen aus der 6. oder 8. Nebengruppe in Lösung mit dem Haftvermittler in Verbindung gebracht, wobei Chelatverbindungen entstehen, die durch ihre Organosiliciumfunktion zur chemischen Fixierung geeignet sind. Anschließend erfolgt die reaktive Anlagerung des edelmetallhaltigen Chelats an die OH-Gruppen des Trägermaterials unter Austritt einer Gruppe X als XH (z.B. HCl, HBr oder $HOR^2$). Dies wird durch 24- bis 100stündiges Erhitzen auf Rückflußtemperatur der in einem unpolaren Lösemittel suspendierten Komponenten erreicht.

Alle weiteren Einzelheiten zu Synthesen ergeben sich aus der Beschreibung der Katalysatorherstellung.

Das Mengenverhältnis von Carbonsäureester bzw. Dialkylether und Jod(verbindung) oder Brom(verbindung) in der Reaktionszone kann innerhalb weiter Grenzen schwanken. Im allgemeinen beträgt die Menge des Carbonsäureesters und/oder Dialkylethers 1 bis 500 mol, vorzugsweise 1 bis 100 mol, je 1 mol Jod(verbindung) oder Brom(verbindung). Die Temperatur der Reaktionszone wird so gewählt, daß das Reaktionsgemisch bei jedem beliebigen Umsatz gasförmig vorliegt. Bevorzugt wird die Temperatur zwischen 150 und 250 °C gewählt. Der bevorzugte Druck liegt zwischen 5 und 30 bar.

Die Verweilzeit des Reaktionsgemisches am festen Trägerkatalysator beträgt 1 bis 1000 s, bevorzugt 1 bis 180 s. Die Umsetzung kann in einem senkrecht angeordneten und mit Trägerkatalysator gefüllten Strömungsrohr oder auch in einem Rühr- oder Schüttelautoklaven erfolgen, der den Trägerkatalysator enthält. Man führt die Carbonylierung im allgemeinen unter wasserfreien Bedingungen durch, doch sind geringe Wassermengen, wie sie in den handelsüblichen Ausgangsstoffen vorkommen, zulässig, sollten aber 1 mol%, berechnet auf die Ausgangsstoffe, nicht übersteigen. Auch wird die Carbonylierung durch geringe Mengen Methanol in den Ausgangsstof fen nicht beeinträchtigt. Ebensowenig stört Wasserstoff, der in kleinen Mengen im handelsüblichen Kohlenmonoxid vorhanden sein kann.

Das aus der Carbonylierungszone abströmende Reaktionsgemisch ist gasförmig und enthält Kohlenmonoxid, Methyljodid, Essigsäureanhydrid, nichtumgesetztes Methlyacetat oder Dimethylether und ggf. sehr geringe Mengen Essigsäure. Das gasförmige Reaktionsgemisch wird abgekühlt, Essigsäureanhydrid und ggf. Essigsäure auskondensiert und die nicht kondensierten Stoffe wie CO, Methyljodid, Methylacetat oder Dimethylether in die Reaktionszone zurückgeführt. Die umgesetzten Anteile von Ester oder Ether sowie CO werden fortlaufend ersetzt.
Die einfache Abtrennung der Anhydride durch Kühlung des abströmenden Reaktionsgemisches und Rückführung der nicht kondensierbaren Gase stellt, wie bei den genannten, bekannten Verfahren, einen wesentlichen Vorteil dar, da dies ohne komplizierte Trennoperationen erfolgen kann. Der Trägerkatalysator wird nicht verunreinigt und verbleibt in der Reaktionszone, was den gesamten Verfahrensablauf bedeutend vereinfacht.

## Beispiele

### Beschreibung der Katalysatorherstellung

In allen Fällen wurden die Katalysatorträger zur Aktivierung zuvor bei 200 °C und 0,1 mbar 10 h getrocknet. Nach Aufbringen der Metallkomponente erhitzte man die Katalysatoren 8 h mit Chlortrimethylsilan zum Sieden und trocknete anschließend bei 0,1 mbar und 100 °C. Sämtliche Synthesen wurden in Argonatmosphäre unter Ausschluß von Luftsauerstoff und Wasser ausgeführt. Alle verwendeten Lösemittel wurden zuvor über Molekularsieb 4 A oder wenn möglich mit Benzophenonnatrium getrocknet.

### Rührautoklavversuche:

Man verwendet einen 0,25 Liter fassenden Rührautoklaven aus korrosionsfreiem Edelstahl (Hastelloy C) mit den erforderlichen Zu- und Ableitungen, der einen drehbaren Katalysatorkorb enthält.
Die Carbonsäureester oder Dialkylether werden gasförmig in Gegenwart des bewegten, festen Trägerkatalysators mit CO-Gas umgesetzt. Der Trägerkatalysator befindet sich in dem drehbaren Katalysatorkorb, der gleichzeitig für die Durchmischung der Gase sorgt.
Der Autoklav wird mit 2,5 ml eines flüssigen Gemisches aus 20 Volumenteilen Methyljodid und 80 Volumenteilen Ester oder Ether beschickt und auf Reaktionstemperatur erhitzt. Die Carbonylierung wird durch Aufpressen von Kohlenmonoxid eingeleitet. Der CO-Druck wird durch regelmäßiges Nachdrücken konstant gehalten.
Die Einzelheiten der Versuchsdurchführung ergeben sich aus den Beispielen.
In den nachfolgenden Beispielen bedeutet Ø = $C_6H_5$-.

### Beispiel 1

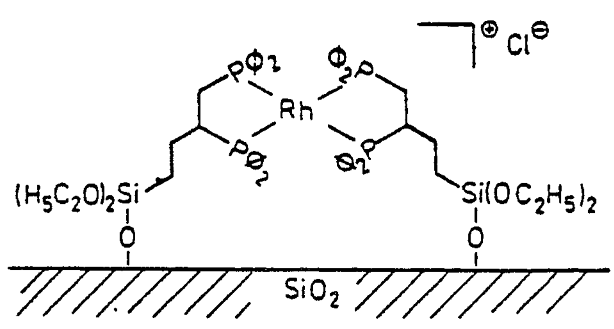

62,9 g aktivierte Siliciumdioxidpellets 1/8" . 1/8" (95 % $SiO_2$) mit einer inneren Oberfläche nach BET von 68 $m^2/g$ und einem Porenvolumen von 0,43 ml/g wurden mit 150 ml einer Lösung aus 722 mg des Komplexes 4 in Toluol versetzt. Die gelbe Suspension wurde 24 h auf Rückflußtemperatur erhitzt, wobei

sich das Lösemittel vollständig entfärbte. Nach Abziehen des Toluols unter reduziertem Druck wurde der Katalysator bei 0,1 mbar und 150 °C 6 h getrocknet und anschließend 24 h in einer Soxhlet-Apparatur mit Benzol extrahiert. Nach der Extraktion konnte im Benzol kein Rhodium nachgewiesen werden.
Charakterisierung: hellgelbe Pellets
Rh-Gehalt: 0,09 Gew.%

Syntheseweg des Rhodiumkomplexes 4

1,2-Dichlor-4-(triethoxysilyl)butan (2):

0,1 mol 1-Butenylmagnesiumbromid (1) in 100 ml Tetrahydrofuran versetzt man tropfenweise mit 0,5 mol Tetraethoxysilan und erhitzt 5 h auf Rückflußtemperatur. Anschließend wird die erhaltene Suspension filtriert und das Lösemittel abgezogen. Den Rückstand nimmt man in Dichlormethan auf und leitet bei 0 °C so lange Chlor ein bis sich die Lösung schwach gelb färbt. Durch Abziehen des Lösemittels und anschließende Vakuumdestillation erhält man 2 in 64 %iger Ausbeute.

1,2-Bis(diphenylphosphino)-4-(triethoxysilyl)-butan (3):

3 wird durch Umsetzung doppeltmolarer Menge Natriumdiphenylphosphid in Dioxan mit 2, gelöst in Tetrahydrofuran, bei Raumtemperatur synthetisiert [analog 1,2-Bis(diphenylphosphino)ethan; siehe K. Issleib und D.-W. Müller, Chem. Ber. 92, 3175 (1959)]. Ausbeute 72 %.

[1,2-Bis(diphenylphosphino)-4-(triethoxysilyl)-butan] rhodium(I)-chlorid (4):

4 mmol 3, gelöst in Benzol, werden unter Rühren zu einer Lösung von 1 mmol Dichlorotetracarbonyldirhodium in Benzol getropft. Abziehen des Lösemittels und Umkristallisieren aus Hexan liefert analysenreinen Komplex 4. Ausbeute 94 %. Vgl. Synthese von [1,2-Bis(diphenylphosphino)-ethan]-rhodium (I)-chlorid; A. Sacco et al. J. Chem. Soc. (London) 3274 (1964).

2 ml (1,86 g) Essigsäuremethylester, 0,5 ml (1,14 g) Methyljodid und 7,2 g des Katalysators werden im Autoklaven mit Kohlenmonoxid bei 15 bar CO-Druck und 180 °C umgesetzt. Nach 1 h Reaktionszeit ergibt sich eine Katalysatorleistung von 19,7 g $Ac_2O/g_{Rh}$ . h, bei einer Selektivität von 95 %.

Ein Stahlrohr von 20 mm Durchmesser und 400 mm Länge wird als Strömungsrohr senkrecht angeordnet und mit 50,6 g des Katalysators gefüllt. Bei einem Druck von 12 bar und einer Temperatur von 180 °C werden stündlich 8 Nl CO (Nl = Liter, gemessen bei 1,013 bar und 0 °C) sowie ein verdampftes Gemisch (12,8 ml Flüssigkeit) aus Methyljodid und Methylacetat (Molverhältnis 1 : 4) durch das Strömungsrohr geleitet.

Das abströmende Reaktionsgemisch wird gaschromatographisch on-line analysiert. Hierbei ergibt sich eine Raum-Zeit-Ausbeute von 12,5 g $Ac_2O/g_{Rh}$ . h, bei einer Selektivität von 97 %.

Unter diesen Reaktionsbedingungen wurde die Carbonylierung 280 Stunden durchgeführt, wobei der eingesetzte Trägerkatalysator keinen Aktivitätsverlust zeigte.

Katalysator Nr. 2

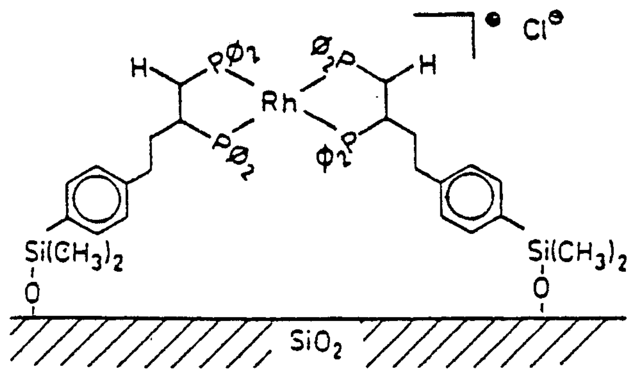

12,7 g aktivierte Siliciumdioxidpellets 1/8" . 1/8" (95 % $SiO_2$) mit einer inneren Oberfläche nach BET von 68 $m^2/g$ und einem Porenvolumen von 0,43 ml/g wurden mit 50 ml einer Lösung aus 133 mg des Komplexes 9 in Toluol versetzt. Die gelbe Suspension wurde 24 h auf Rückflußtemperatur erhitzt, wobei sich das Lösemittel vollständig entfärbte. Nach Abziehen des Toluols unter reduziertem Druck wurde der Katalysator bei 0,1 mbar und 150° C 6 h getrocknet und anschließend 24 h in einer Soxhlet-Apparatur mit Benzol extrahiert. Nach der Extraktion konnte im Benzol kein Rhodium nachgewiesen werden.
Charakterisierung: hellgelbe Pellets
Rh-Gehalt: 0,08 Gew.%

Syntheseweg des Rhodiumkomplexes 9

1,2-Dichlor-4-(4-chlorphenyl)butan (6):

6 kann durch Umsetzung von 4-(4-Chlorphenyl)buten (5) mit Chlor bei 0° C in Dichlormethan synthetisiert werden. Ausbeute 93 %.

1,2-Bis(diphenylphosphino)-4-(4-chlorphenyl)-butan (7):

7 wird durch Umsetzung doppeltmolarer Menge Natriumdiphenylphosphid in Dioxan mit 6, gelöst in Tetrahydrofuran, bei Raumtemperatur mit 82 % Ausbeute synthetisiert [analog 1,2-Bis(diphenylphosphino)-ethan; siehe K. Issleib und D.-W. Müller, Chem. Ber. 92, 3175 (1959)].

1,2-Bis(diphenylphosphino)-4-[4-(dimethylethoxysilyl)-phenyl]butan (8) :

0,05 mol 7 werden in Tetrahydrofuran zur Arylmagnesiumchloridverbindung umgesetzt; siehe R. D. Rieke u. S. E. Bales, J. Am. Chem. Soc. 96, 1775 (1974); J. P. Collman et al. J. Am. Chem. Soc. 105, 7288 (1983). Anschließend tropft man unter Rühren und Eiskühlung 0,25 mol Diethoxydimethylsilan zu, läßt auf Raumtemperatur erwärmen und erhitzt schließlich 5 h auf Rückflußtemperatur. Die Reaktionsmischung wird filtriert; Lösemittel und überschüssiges Diethoxydimethylsilan werden im Vakuum abgezogen. Den öligen Rückstand kristallisiert man aus Hexan und erhält 8 in 68 %iger Ausbeute.

[1,2-Bis(diphenylphosphino)-4-[4-(dimethylethoxysilyl) phenyl]butan] rhodium(I)-chlorid (9):

4 mmol 8, gelöst in Benzol, werden unter Rühren zu einer Lösung von 1 mmol Dichlorotetracarbonyldirhodium in Benzol getropft. Abziehen des Lösemittels und Umkristallisieren aus Hexan liefert analysenreinen Komplex 9. Ausbeute 95 % Vgl. Synthese von [1,2-Bis(diphenylphosphino)-ethan]-rhodium (I)-chlorid; A. Sacco et al., J. Chem. Soc. (London) 3274 (1964).

2 ml (1,86 g) Essigsäuremethylester, 0,5 ml (1,14 g) Methyljodid und 7,9 g des Katalysators werden im Autoklaven mit Kohlenmonoxid bei 15 bar CO-Druck und 180° C umgesetzt. Nach 1 h Reaktionszeit ergibt

sich eine Katalysatorleistung von 18,8 g $Ac_2O/g_{Rh}$ . h bei einer Selektivität von 96 %

Katalysator Nr. 3

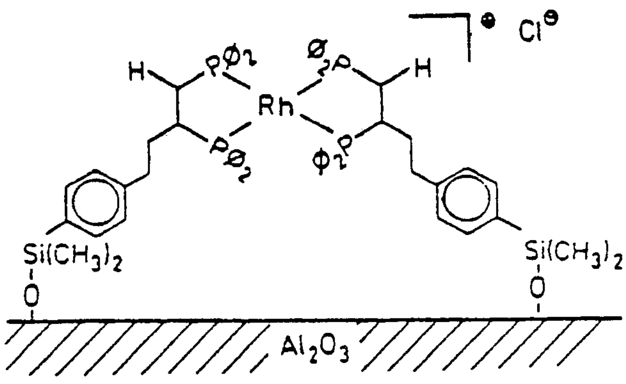

11,7 g aktivierte Aluminiumoxidkugeln (99 % $Al_2O_3$) mit einem Durchmesser von 3 mm, einer inneren Oberfläche nach BET von 125 $m^2/g$ und einem Porenvolumen von 0,9 ml/g wurden mit 50 ml einer Lösung aus 156 mg des Komplexes 9 in Toluol versetzt. Die gelbe Suspension wurde 24 h auf Rückflußtemperatur erhitzt, wobei sich das Lösemittel vollständig entfärbte. Nach Abziehen des Toluols unter reduziertem Druck wurde der Katalysator bei 0,1 mbar und 150°C 6 h getrocknet und anschließend 24 h in einer Soxhlet-Apparatur mit Benzol extrahiert. Nach der Extraktion konnte im Benzol kein Rhodium nachgewiesen werden.
Charakterisierung: hellgelbe Kugeln
Rh-Gehalt: 0,1 Gew.%

2 ml (1,86 g) Essigsäuremethylester, 0,5 ml (1,14 g) Methyljodid und 6,5 g des Katalysators werden im Autoklaven mit Kohlenmonoxid bei 15 bar CO-Druck und 180°C umgesetzt. Nach 1 h Reaktionszeit ergibt sich eine Katalysatorleistung von 41,0 g $Ac_2O/g_{Rh}$ . h, bei einer Selektivität von 89 %.

**Ansprüche**

1. Trägerkatalysator für die Herstellung von Monocarbonsäureanhydriden durch Carbonylierung der entsprechenden Ester oder Ether, worin eine Organosiliciumverbindung mit Alkoxy- oder Halogengruppen sowie mit Organostickstoff-, Organophosphor-, Organoarsen-, Organoschwefel- oder Mercaptogruppen als mehrfunktioneller Haftvermittler einerseits an ein Trägermaterial und andererseits an eine Edelmetallverbindung aus der Gruppe VIII des Periodensystems der Elemente gebunden ist, dadurch gekennzeichnet, daß der Haftvermittler eine chelatbildende Organosiliciumverbindung der allgemeinen Formel

a) 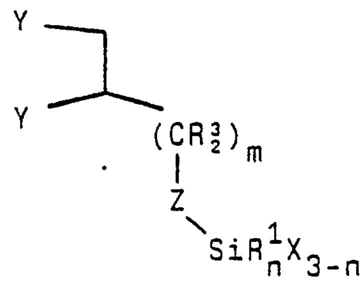 oder

b) 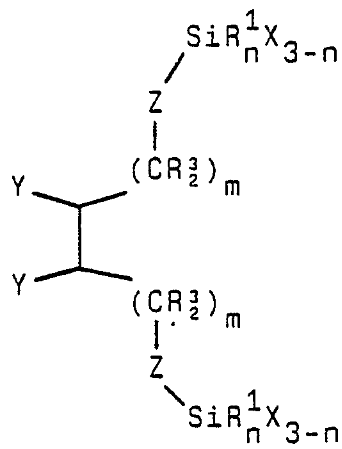

ist, wobei
X = -Cl, -Br oder $-OR^2$;
Y = $-NR_2^4$, ein stickstoffhaltiger Arylrest, $-PR_2^4$, $AsR_2^4$, $-SR^4$ oder -SH;
Z = null, Arylen, Phenylen (ggf. ortho-, meta- oder parasubstituiert)
$R^1$ = $C_1$ bis $C_5$-Alkyl;
$R^2$ = $C_1$ bis $C_5$-Alkyl oder $-C_6H_5$; $R^3$ = -H, $C_1$ bis $C_3$-Alkyl; $R^4$ = $C_1$ bis $C_6$-Alkyl, $C_5$ oder $C_6$-Cycloalkyl oder $-C_6H_5$ oder $-CH_2C_6H_5$, die ggf. mit Halogen-, Methoxy-, Ethoxy- oder $C_1$ bis $C_3$-Alkylgruppen substituiert sind;
n = 0 oder 1 oder 2;
m = 2 bis 6, vorzugsweise 2 bis 4.

2. Trägerkatalysator nach Anspruch. 1, dadurch gekennzeichnet, daß in ihm die chelatbildende Organosiliciumverbindung als mehrfunktioneller Haftvermittler einerseits an das Trägermaterial und andererseits abwechselnd an die Edelmetallverbindung und an eine Nichtedelmetallverbindung aus der 6. oder 8. Nebengruppe des Periodensystems gebunden ist.

3. Trägerkatalysator nach Anspruch 1 oder 2, dadurch gekennzeichnet, daß er zusätzlich Nichtedelmetallverbindungen aus der 1. bis 3. Hauptgruppe oder der 4. bis 6. oder der 8. Nebengruppe des Periodensystems als Promotoren enthält.

4. Trägerkatalysator nach einem der Ansprüche 1-3, dadurch gekennzeichnet, daß er ein anorganisches oxidisches Trägermaterial oder einen Aktivkohleträger enthält, deren restliche aktive Hydroxygruppen durch Veresterung oder Veretherung desaktiviert wurden.

5. Trägerkatalysator nach einem der Ansprüche 1-4, dadurch gekennzeichnet, daß er zusammen 0,01 bis 50 Gew%, vorzugsweise 0,1 bis 20 Gew%, Edelmetallverbindung, Haftvermittler und ggf. Nichtedelmetallverbindung enthält.

6. Trägerkatalysator nach einem der Ansprüche 1-5, gekennzeichnet durch die Formel

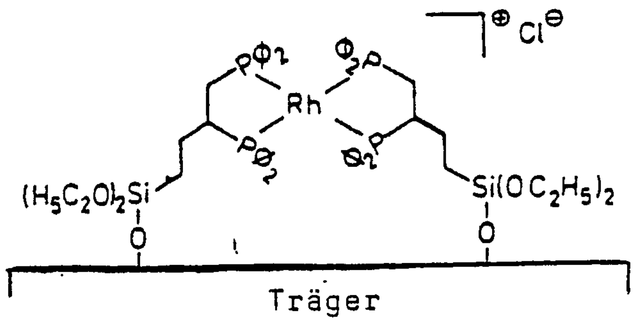

7. Trägerkatalysator nach einem der Ansprüche 1-5, gekennzeichnet durch die Formel

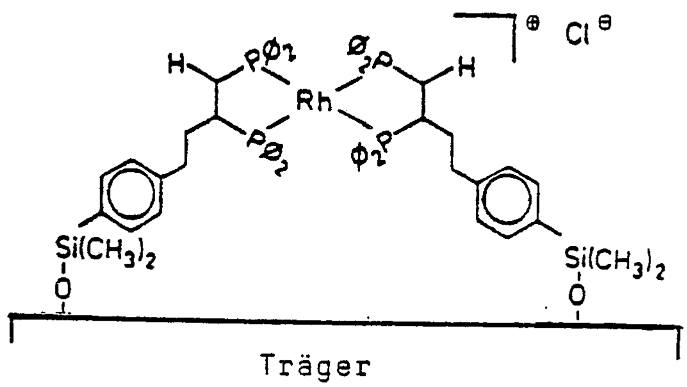

Europäisches Patentamt

# EUROPÄISCHER RECHERCHENBERICHT

Nummer der Anmeldung

EP 89 10 3879

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 180 802 (HOECHST)<br>* Ansprüche *<br>--- | 1-7 | B 01 J 31/16<br>C 07 C 51/56 |
| Y | US-A-4 542 119 (C.-Y. HSU et al.)<br>* Anrpuch 1 *<br>--- | 1-7 | |
| A | EP-A-0 263 564 (SHELL)<br>--- | | |
| A | EP-A-0 185 882 (DEGUSSA)<br>--- | | |
| A | EP-A-0 245 893 (SHELL)<br>--- | | |
| A | GB-A-2 092 017 (NEFTEKHIMICHESKOGO)<br>--- | | |
| A | EP-A-0 079 461 (HOECHST)<br>----- | | |

| RECHERCHIERTE SACHGEBIETE (Int. Cl.4) |
|---|
| B 01 J<br>C 07 C |

Der vorliegende Recherchenbericht wurde für alle Patentansprüche erstellt

| Recherchenort | Abschlußdatum der Recherche | Prüfer |
|---|---|---|
| DEN HAAG | 22-06-1989 | THION M.A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)